(19) Europäisches Patentamt
European Patent Office
Office européen des brevets

(11) Publication number: **0 129 214 B1**

(12) # EUROPEAN PATENT SPECIFICATION

(45) Date of publication of the patent specification: **29.10.86**

(51) Int. Cl.⁴: **C 07 C 85/20**, C 07 C 87/60, C 07 C 125/00

(21) Application number: **84106805.9**

(22) Date of filing: **14.06.84**

(54) **Method for the preparation of benzenamines.**

(30) Priority: **15.06.83 US 504636**
**15.06.83 US 504637**

(43) Date of publication of application:
**27.12.84 Bulletin 84/52**

(45) Publication of the grant of the patent:
**29.10.86 Bulletin 86/44**

(84) Designated Contracting States:
**DE FR GB IT NL**

(56) References cited:
**AT - B - 340 946**
**DE - B - 1 543 624**
**GB - A - 955 898**
**US - A - 3 829 460**

**L. FIESER UND M. FIESER "Organische Chemie", 1965, VERLAG CHEMIE GMBH, Weinheim, page 605**
**ANGEWANDTE CHEMIE, vol. 78, 1966, Weinheim, E. KLAUKE "Herstellung und Eigenschaften von Substanzen mit an Stickstoff oder Schwefel gebundenen Perhalogenmethylgruppen", page 829**

(73) Proprietor: **OCCIDENTAL CHEMICAL CORPORATION, P.O. Box 189, Niagara Falls New York 14302 (US)**

(72) Inventor: **Lin, Henry C., 1971 Huth Road, Grand Island, NY 14072 (US)**
Inventor: **Cotter, Byron R., 450 Crest Drive, Northvale, NJ 07647 (US)**

(74) Representative: **Vossius & Partner, Siebertstrasse 4 P.O. Box 86 07 67, D-8000 München 86 (DE)**

EP 0 129 214 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a method for the preparation of benzenamines and to the preparation of phenyl carbamic fluorides, useful in the preparation of benzenamines.

The benzenamines, also known as anilines and as aminobenzenes, are a known class of compounds having commercial utility as chemical intermediates, for a wide range of chemical end products. Substituted benzenamines, especially trifluoromethyl-substituted benzenamines such as 2-(trifluoromethyl)-benzenamines (also known as o-trifluoromethyl anilines or o-aminobenzotrifluorides) are particularly useful as intermediates for the preparation of various dyestuff and pesticides. For example, 2-amino-5-chlorobenzotrifluoride [also known as 4-chloro-2-(trifluoromethyl)benzenamine], is employed commercially as a dye intermediate and is designated as C.I. Azoic Diazo Component 17, according to Colour Index, Chemical No. 37055, Vol. 1-4, 2nd ed. 1956, Suppl. 1963 published by the Society of Dyers and Colourists (U.K.) and The American Association of Textile Chemists and Colorists (U.S.).

Substituted benzenamines, such as (trifluoromethyl)-benzenamines are disclosed in U.S. Patent 4 243 819 to Henrick et al., as intermediates in the synthesis of amino acid esters having pesticidal properties. Thus, for example, the reference teaches the preparation of such esters by reaction of various trifluoromethylanilines with m-phenoxybenzyl α-bromoisovalerate.

U.S. Patent 4 316 988 to Clinton discloses the use of trifluoromethyl-substituted anilines as intermediates in the synthesis of various diphenylamine products useful as rodenticides, insecticides, and arachnicides.

The utility of benzenamines, including o-aminobenzotrifluorides as chemical intermediates has led to the investigation and development of various methods for the preparation of these compounds.

One known method for the preparation of aromatic amines, such as aniline, involves the reduction of an aromatic nitro compound. Thus, aniline may be prepared by reaction of nitrobenzene with hydrogen. McBee et al., J. Am. Chem. Soc. 73, 3932-34 (1951) disclose the preparation of 4-bromo-2-(trifluoromethyl)-aniline by nitration of 3-bromo-(trifluoromethyl)-benzene followed by reduction of the resultant 2-nitro-5-bromo(trifluoromethyl)-benzene.

U.S. Patent 4 096 185 to Seiwell discloses the preparation of p-aminobenzotrifluoride [also known as 4-(trifluoromethyl)-benzenamine] by reaction of p-chlorobenzotrifluoride with ammonia in a nonaqueous solvent in the presence of copper compound, such as cuprous chloride and a selected salt, such as potassium fluoride.

Forbes et al., Tetrahedron, Vol. 8, 67-72 (1960) prepared o-aminobenzotrifluoride by hydrogenation of 2-nitrotrifluoromethylbenzene at elevated temperatures and pressure in the presence of a Raney nickel catalyst.

German Offenlegungsschrift D.E. 3 017 542 to Klauke et al., discloses the preparation of o-aminobenzotrifluoride by hydrogenation-hydrogenolysis of 2-amino-5-chlorobenzotrifluoride.

It is also known that isocyanates and carbamic acid fluorides are susceptible to hydrolysis, in a known manner, to form an amine. See Fieser and Fieser, Organic Chemistry, 3rd Ed D.C. Health and Co. (1956). However, when the reaction is run neat, substantial quantities of urea may be formed as by-product. The formation of urea may be minimized, or avoided when a solvent, such as toluene, is employed, but the yields of amine are low.

Although methods for the preparation of aromatic amines, such as aminobenzotrifluorides are known from the prior art, it will be appreciated that the development of improved and more economical processes is desirable.

The present process for the preparation of benzenamines differs substantially from the prior art process in the use of hydrogen fluoride and phenyl carbamic fluorides as reactants. Furthermore, the preparation of 2-(trifluoromethyl)phenyl carbamic fluorides in accordance with this invention, differs substantially from the prior art processes. The preparation of the phenyl carbamic fluorides by reaction of phenyl isocyanates and hydrogen fluoride is known. Buckley et al. J. Chem. Soc. 864 (1945) disclose the preparation of phenyl carbamic acid fluorides by reaction of HF with various isocyanates. However, the reference provides no teaching relative to the fluorination of aromatic isocyanates bearing a haloalkyl side chain and no teaching or suggestion of the preparation of aromatic amines. The reference further discloses the treatment of phenylcarbamyl fluoride with water to result in the formation of diphenylurea.

British Patent 955 898 (1964) to Farbenfabriken Bayer Aktiengesellschaft discloses the reaction of anhydrous hydrogen fluoride with chloromethylphenyl isocyanates to produce the corresponding fluoromethylphenyl carbamic acid fluoride or, upon subsequent heating, the corresponding isocyanate. The British patent further discloses the reaction of hydrogen fluoride with 2-trichloromethyl-4-chlorophenyl isocyanate in chlorerobenzene to prepare 2-trifluoromethyl-4-chlorophenyl carbamic acid fluoride. Subsequently, Klauke, Angew. Chem. Interat. Ed. Vol. 5, No. 9, 848, (1966), in contrast to the teachings of Brit. Pat. 955 898, stated that when o-trichloromethylphenyl isocyanate undergoes chlorine-fluorine exchange in anhydrous hydrogen fluoride; isomerization occurs simultaneously and o-N-(trifluoromethyl)aminobenzoyl fluoride can be isolated, thus implying that o-trifluoromethyl phenyl carbamic acid fluoride is not produced. In U.S. Patent 3 829 460 to Buttner and Klauke assigned to Bayer Aktiengesellschaft, reference is made to the 1966 article and to earlier contradictory teachings and it is disclosed that when hydrogen fluoride is reacted with a trichloromethylphenyl isocyanate wherein the trichloromethyl group is in the 2-position to the isocyanate group, it is only possible to obtain the isomers, 2-N-trifluoromethylamino-benzoyl fluorides.

British Patent 1 164 223 to Klauke et al. teaches the hydrolysis of trifluoromethylphenyl isocyanates

with 90-100 percent sulfuric acid to produce the corresponding trifluoromethyl benzenamine (or aminosulfate) with carbon dioxide as the only by-product.

The hydrolysis of an NCO group with acid agents, such as concentrated hydrochloric acid or sulphamic acid is known (Houben-Weyl, Methoden der org. Chemie, 4th Edition, Vol. 11/1, page 953).

Although the utility and commercial value of aromatic amines has been generally recognized for many years; and, various investigations have been made of reactions of aromatic isocyanates, including aromatic isocyanates, including aromatic isocyanates having a perchlorinated alkyl side chain, there has been no suggestion heretofore that aromatic amines can be prepared by the reaction of hydrogen fluoride with phenyl carbamic acid fluorides or their precursor phenyl isocyanates. Furthermore, it has not been known heretofore that 2-(trifluoro-methyl)phenyl carbamic fluorides may be conveniently prepared by the isomerization of N-(trifluoromethyl)-anthraniloyl fluorides of hydrogen fluoride.

*SUMMARY OF THE INVENTION*

It has now been found that benzenamines, of the formula

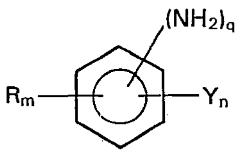

wherein R is chlorine, fluorine, bromine, phenyl, chlorophenyl, fluorophenyl, or bromophenyl
Y is trifluoromethyl or difluoromethyl
m is 0 to 2
n is 0 to 2
q is 1 to 2
can be prepared by

(A) reacting hydrogen fluoride with a phenyl carbamic fluoride of the formula

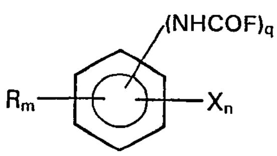

where q, m, n, and R are as defined above, and X is trichloromethyl, tribromomethyl, trifluoromethyl, dichloromethyl, dibromomethyl or difluoromethyl with the provision that when X is trichloromethyl, tribromomethyl or trifluoromethyl, Y is trifluoromethyl and when X is dichloromethyl, dibromomethyl or difluoromethyl, Y is difluoromethyl, to produce a benzenamine·hydrofluoride complex; of the formula

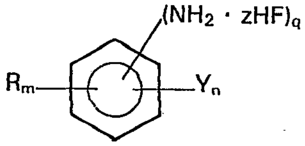

where z is about 1 to about 4 and R, Y, q, m and n are as defined above; and

(B) dissociating the benzenamine·hydrofluoride complex and recovering the benzenamine.

The benzenamine·hydrofluoride resulting from the reaction of hydrogen fluoride with the phenyl carbamic fluoride in accordance with step (A), above, is a complex of variable stoichiometry. The explanation of the variable stoichiometry of these benzenamine·hydrofluoride complexes is not essential to the process of this invention or the preparation or use of such complexes. However, it may be postulated that the variable stoichiometry is a result of hydrogen bonding.

The amine·HF complex can be dissociated by heating for example, to temperatures above 100° Celsius. However, at such temperatures the dissociation may be accompanied by undesirable reactions such as polymerization. In a prefered manner, the benzenamine·hydrofluoride complex can be readily dissociated by conventional methods such as neutralization with caustic or the like, and the benzenamine recovered from the reaction mixture by conventional physical separation processes such as distillation or the like. The neutralization of amine·hydrofluorides with KOH, NaOH or the like is disclosed by Berliner et al., Journal of Physical Chemistry, Vol. 32, 1142-1162, (1928).

Generally for the reaction of hydrogen fluoride with the phenyl carbamic fluoride, a temperature in the range of about —10° to about 150° Celsius and preferably about 20° to about 100° Celsius is employed. The reaction may be run neat or in the presence of a carrier medium, such as methylene chloride. The reaction proceeds smoothly, without the need for a catalyst. However, if desired, a catalyst, such as a Lewis acid catalyst may be employed.

The reaction of the phenyl carbamic acid fluoride with hydrogen fluoride is carried out in the presence of a stoichiometric excess of hydrogen fluoride to result in the formation of the benzenamine·hydrofluoride and carbonyl fluoride as a by-product. The amount of hydrogen fluoride provided to the reaction should be at least slightly in excess of the amount required for the formation of the benzenamine·hydrofluoride. Preferably the amount of hydrogen fluoride will be sufficient to provide a molar ratio of hydrogen fluoride:phenyl carbamic fluoride of between 4:1 and 30:1 or greater. It is preferred to carry out the reaction in the liquid phase either in a sealed reactor, that is, an autoclave, or at atmospheric pressure, using a cooling condenser.

The phenyl carbamic fluoride reactant may be conveniently prepared by reaction of the corresponding phenyl isocyanate with hydrogen fluoride, The reaction may be carried out in the liquid or vapor phase. In the liquid phase the reaction may be carried out at atmospheric pressure, with the temperature being maintained at below the boiling point of hydrogen fluoride, or at higher pressures and temperatures under autogenous conditions. It is preferred to carry out the reaction neat. However, if desired, a carrier medium such as methylene chloride may be

employed. Typically, for a liquid phase reaction, temperatures in the range of about 20° to about 100° Celsius are employed. In a vapor phase reaction the temperatures will generally be above the boiling point of the reaction mixture with no practical upper limit. Typically, the vapor phase reaction is run at a temperature of about 250° to about 350° Celsius. It is preferred to carry out the reaction with at least slight stoichiometric excess of hydrogen fluoride present at all times. Altough there is no practical upper limit to the molar ratio of hydrogen fluoride to organic reaction employed, a ratio between about 5:1 and about 25:1 is generally employed.

It has been found that when hydrogen fluoride is reacted with a 1-isocyanato-2-(trihalomethyl)benzene to prepare a 2-(trifluoromethyl)phenyl carbamic fluoride, the in situ formation of N-(trifluoromethyl)-anthraniloyl fluoride occurs during the reaction. The anthraniloyl fluoride may be isolated and recovered. However, if left under reaction conditions, a rearrangement or isomerization of the N-(trifluoromethyl)phenyl carbamic fluoride is formed. This intermediate formation of an N-(trifluoromethyl)anthraniloyl fluoride and the isomerization thereof has only been found in reactions involving orthotrihalomethyl-substituted phenyl isocyanates, such as 1-isocyanato-2-(trihalomethyl)benzene and ring substituted derivatives, and does not appear to occur in reactions involving meta- or para-trihalomethyl-substituted phenyl isocyanates.

Thus, in accordance with another aspect of the present invention, it has been found that 2-(trifluoromethyl)phenyl carbamic fluorides of the formula

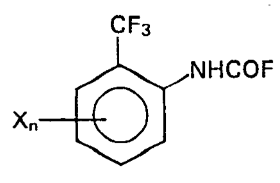

where n is 0 to 2 and X is independently selected from the group fluoro-, chloro-, bromo-, phenyl-, chlorophenyl-, bromophenyl-, and fluorophenyl- can be prepared by isomerizing an N-(trifluoromethyl)-anthraniloyl fluoride of the formula

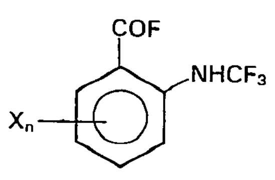

where n and X are as defined above, in the presence of hydrogen fluoride.

The isomerization of N-(trifluoromethyl)-anthraniloyl fluoride to 2-(trifluoromethyl)phenyl carbamic fluoride proceeds readily in the presence of hydrogen fluoride. Preferably, the hydrogen fluoride is present in an amount of about 1.5 to about 25 and most preferably about 4.0 to about 20.0 moles of hydrogen fluoride per mole of N-(trifluoromethyl)-anthraniloyl fluoride. When lesser amounts are employed the isomerization will occur, but the reaction time is much longer. Greater amounts of hydrogen fluoride may be employed, but provide no special advantage.

It is preferred to carry out the isomerization in the liquid phase. The process may be carried out at atmospheric pressure, with the temperature being maintained at or below the boiling point of hydrogen fluoride, or at higher pressures and temperatures under autogenous conditions. Temperatures, for example, in the range of about —10° or less to about 150° Celsius or higher may be employed. Preferably, temperatures in the range of about 20° to about 100° Celsius are employed.

The reaction may be run neat or in the presence of a carrier medium such as methylene chloride. The reaction proceeds smoothly without the need for a catalyst. However, if desired, a catalyst, such as a Lewis acid catalyst, such as antimony pentachloride, may be employed.

The reactants susceptible to isomerization in accordance with this invention include N-(trifluoroethyl)-anthraniloyl fluoride as well as the various derivatives bearing ringsubstituents, such as, fluoro-, chloro-, bromo-, phenyl-, chlorophenyl-, fluorophenyl-, or bromophenyl-. The isomerization of N-(trifluoromethyl)-anthraniloyl fluoride to 2-(trifluoromethyl)-phenyl carbamic fluoride is of particular interest due to the utility of the latter as an intermediate for the subsequent preparation of 2-(trifluoromethyl)-benzenamine.

N-(trifluoromethyl)-anthraniloyl fluoride may be conveniently prepared by the reaction of 1-isocyanato-2-(trichloromethyl)-benzene. The reaction may be carried out in the liquid or vapor phase. In the liquid phase the reaction may be carried out at atmospheric pressure, with the temperature being maintained at or below the boiling point of hydrogen fluoride, or at higher pressures and temperatures under autogenous onditions. Typically for a liquid phase reaction, temperatures in the range of about —10° to about 100° Celsius are employed. In a vapor phase reaction the temperature will generally be above the boiling point of the reaction mixture with no practical upper limit. Typically, the vapor phase reaction is run at a temperature of about 250° to about 350° Celsius.

It is preferred to carry out the reaction of the 1-isoyanato-2-(trichloromethyl)-benzene and hydrogen fluoride with at least a slight stoichiometric excess of hydrogen fluoride present at all times. Although there is no theoretical upper limit to the molar ratio of hydrogen fluoride to organic reaction employed, a ratio between about 4:1 and about 25:1 is generally employed. The reaction may be run neat or in the presence of a carrier medium such as methylene chloride.

The N-(trifluoromethyl)-anthraniloyl fluoride reactant may be employed in substantially pure form or, conveniently, as the crude reaction product of hydrogen fluoride and 1-isocyanato-2-(trichloromethyl)-benzene as described in the preceding paragraph. Thus, in one embodiment, the present invention provides a process for the preparation of 2-(trifluoromethyl)-phenyl carbamic fluoride comprising (A) reacting 1-isocyanato-2-(trichloromethyl)-benzene with hydrogen fluoride to form N-(trifluoromethyl)-anthraniloyl fluoride; and (B) isomerizing the N-(trifluoromethyl)-anthraniloyl fluoride in the presence of hydrogen fluoride to form 2-(trifluoromethyl)phenyl carbamic fluoride.

The 2-(trifluoromethyl)phenyl carbamic fluoride resulting from the isomerization step of this invention may be recovered from the crude reaction product in relatively pure form by conventional physical separation means, such as distillation. Alternatively, if the 2-(trifluoromethyl)phenyl carbamic fluoride is to be employed as a chemical intermediate for the preparation of 2-(trifluoromethyl)-benzenamine, or the hydrofluoride salt thereof, it may be employed in the crude form, without purification or separation from the reaction mixture. In this instance, the crude 2-(trifluoromethyl)phenyl carbamic fluoride product of the isomerization step is further reacted with hydrogen fluoride to form the hydrofluoride salt or complex of 2-(trifluoromethyl)-benzenamine.

Thus, the phenyl carbamic fluoride reactant may be employed in substantially pure form or as the crude product of the reaction of hydrogen fluoride and a phenyl isocyanate prepared as described above. Thus, in one aspect, this invention provides a process for the preparation of benzenamine which comprises reacting a phenyl isocyanate with hydrogen fluoride to form a phenyl carbamic acid fluoride and continuing the reaction of the phenyl carbamic acid fluoride with hydrogen fluoride to produce the benzenamine.

In another aspect of the present invention, it has been found surprisingly that the reaction of a phenyl carbamic fluoride with hydrogen fluoride to form the benzamine hydrofluoride, may be accelerated by the addition of water to the reaction mixture. The amount of water added to the reaction mixture is not critical and will be effective even in trace amounts. Typically, the amount of water employed will be in the range of about 0.001 to about 2.0 moles of water per mole of phenyl carbamic fluoride. Thus, in a preferred embodiment of this invention, the reaction of hydrogen fluoride with phenyl carbamic fluoride is carried out in the presence of 4 to about 30 moles of hydrogen fluoride per mole of phenyl carbamic fluoride, and in the further presence of about 0,001 to about 2.0 moles of water per mole of phenyl carbamic fluoride to form the corresponding benzenamine·hydrofluoride.

The following specific examples are provided to further illustrate this invention and the manner in which it may be carried out. It will be understood, however, that the specific details given in the examples have been chosen for purpose of illustration and are not to be construed as a limitation on the invention. In the examples, unless otherwise indicated, all parts and percentages are by weight and all temperatures are in degrees Celsius.

*Examples 1-6*

*Preparation of 2-(Trifluoromethyl)Benzenamine from 2-(Trifluoromethyl)Phenyl Carbamic Fluoride*

*Example 1*

Three hundred and two parts of 2-(trifluoromethyl)phenyl carbamic fluoride was charged to a polytetrafluoroethylene reaction vessel and maintained at 0°C while 420 parts of liquid hydrogen fluoride was added. The reaction vessel was then sealed and the temperature was increased to about 20°C and maintained thereat, with agitation, for about 16 hours. The reaction vessel was then opened to atmospheric pressure. Analysis of the organic liquid reaction product by gas chromatographic techniques indicated 54.6% 2-(trifluoromethyl)phenyl carbamic fluoride starting material.

*Example 2*

2-(Tifluoromethyl)phenyl carbamic fluoride was prepared in situ by the reaction of 187 parts of 1-isocyanato-2-(trifluoromethyl)benzene and 336 parts of hydrogen fluoride added over a 15 minute period at 0°C. Eighteen parts of water was added to the 2-(trifluoromethyl)phenyl carbamic fluoride reaction product and the temperature was increased to about 60°C. The temperature was maintained thereat while hydrogen fluoride was continually refluxed and returned to the reaction mixture. After 24 hours, analysis of the liquid reaction mixture, using gas chromatographic techniques, indicated that the organic reaction product contained 99 percent 2-(trifluoromethyl)benzenamine and 1.0 percent 2-(trifluoromethyl)phenyl carbamic fluoride.

The liquid reaction product is made alkaline by addition of NaOH and distilled under reduced pressure to recover 2-(trifluoromethyl)benzenamine.

*Examples 3-6*

The procedure of Example 2 was repeated except that conditions were varied as shown, with the results as set forth in Table 1, below.

TABLE 1

| Example | Temperature (°C) | Pressure Conditions | HF (Parts) | $H_2O$ (Parts) | Organic Product Composition (%) 2-(trifluoromethyl)benzenamine | 2-(trifluoromethyl)phenyl carbamic fluoride |
|---|---|---|---|---|---|---|
| 2 | 60 | Atmospheric[1] | 336 | 18 | 99 | 1 |
| 3 | 25 | Atmospheric[1] | 355 | 18 | 63.3 | 36.7 |
| 4 | 25 | Autogenous | 338 | 18 | 54.9 | 45.1 |
| 5 | 60 | Atmospheric[1] | 355 | 0 | 49.9 | 50.1 |
| 6 | 25 | Autogenous | 394 | 0 | 22.2 | 77.1 |

[1]HF Reflux Conditions

*Example 7*

*Preparation of 2-(Trifluoromethyl)Benzenamine*

2-(Trifluoromethyl)phenyl carbamic fluoride was prepared in situ by the reaction of 515 parts of 1-isocyanato-2-(trichloromethyl)benzene and 1015 parts of hydrogen fluoride added over a period of about 2 hours at a temperature of about 0°-4°C. The

reaction mixture was then sealed in a polytetrafluoroethylene reactor heated to about 24°C and maintained thereat, with agitation, for about 48 hours. The reactor was then opened and equipped with a cooling condenser. Thirty-nine parts of water was added and the reaction mixture was stirred at atmospheric pressure for about 48 hours. The reactor was then opened and equipped with a cooling condenser. Thirty-nine parts of water was added and the reaction mixture was stirred at atmospheric pressure for 120 hours. The reaction mixture was then swept with nitrogen to remove hydrogen fluoride, and rendered alkaline by slow addition of 305 parts of 20% aqueous sodium hydroxide, washed with methylene chloride, then distilled under reduced pressure to yield 293 parts of 2-(trifluoromethyl)benzenamine.

*Example 8*

*Preparation of 3-(Trifluoromethyl)Benzenamine*

3-(Trifluoromethyl)phenyl carbamic fluoride was prepared in situ by the reaction of 187 parts of 1-isocyanato-3-(trifluoromethyl)benzene and 370 parts of hydrogen fluoride added over a period of about 15 minutes at about 4°C. The reaction mixture was then sealed in a polytetrafluoroethylene reaction vessel, heated to about 24°C, and maintained thereat, with agitation, for about 96 hours. The reaction vessel was then opened to atmospheric pressure and most of the hydrogen fluoride and carbonyl fluoride gases removed. The remaining organic product was analyzed by gas chromatographic techniques, using n-pentadecane as an internal standard, indicating a yield of 41 parts of 3-(trifluoromethyl)benzenamine.

*Example 9*

*Preparation of 4-Chloro-2-(Trifluoromethyl)Benzenamine*

4-Chloro-2-(trifluoromethyl)phenyl carbamic fluoride was prepared in situ by the reaction of 221 parts of 4-chloro-2-(trifluoromethyl)-isocyanatobenzene and 380 parts of hydrogen fluoride added over a period of about 15 minutes at about 4°C. The reaction mixture was then sealed in a polytetrafluoroethylene reactor, heated to about 24°C and maintained at about that temperature, with agitation, for about 96 hours. The reactor was then opened to atmospheric pressure and most of the hydrogen fluoride and carbonyl fluoride gases removed. Analysis of the remaining organic product by gas chromatographic techniques, using n-pentadecane as an internal standard, indicated a yield of 55 parts of 4-chloro-2-(trifluoromethyl)benzenamine.

*Example 10*

*Preparation of 4-Chloro-3-(Trifluoromethyl)Benzenamine*

4-Chloro-3-(trifluoromethyl)phenyl carbamic fluoride was prepared in situ by the reaction of 221 parts of 4-chloro-3-(trifluoromethyl)isocyanatobenzene and 450 parts of hydrogen fluoride added aver a period of 15-20 minutes at about 4°C. The reaction mixture was then sealed in a polytetrafluoroethylene reactor, heated to about 24°C and maintained thereat, with agitation, for about 96 hours. The reactor was then opened to atmospheric pressure and most of the hydrogen fluoride and carbonyl fluoride gases removed. Analysis of the remaining organic product by gas chromatographic techniques, using n-heptadecane as an internal standard indicated a yield of 77 parts of 4-chloro-3-(trifluoromethyl)benzenamine.

*Example 11*

*Preparation of 4-(Trifluoromethyl)Benzenamine*

4-(Trifluoromethyl)phenyl carbamic fluoride was prepared in situ by the reaction of 224 parts of 1-isocyanato-4-(trifluoromethyl)benzene and 460 parts of hydrogen fluoride added over a period of about 35 minutes at about 3°C. The reaction mixture was then sealed in a polytetrafluoroethylene reaction vessel, heated to about 24°C, and maintained thereat, with agitation, for about 112 hours. Infrared analysis of the gaseous phase, when the reaction vessel was opened to atmospheric pressure, indicated large quantities of carbonyl fluoride present. The remaining organic product was analyzed by gas chromatographic techniques, using n-pentadecane as an internal standard, indicating a yield of 114 parts of 4-(trifluoromethyl)benzenamine.

*Example 12-16*

*Isomerization of N-(Trifluoromethyl)-Anthraniloyl Fluoride to 2-(Trifluoromethyl) Phenyl Carbamic Fluoride*

*Example 12*

Three parts of N-(trifluoromethyl)-anthraniloyl fluoride was charged to a polytetrafluoroethylene reactor. The reactor was maintained at 0°C while three parts of liquid hydrogen fluoride was added. The reactor was then sealed and the temperature was increased to about 25°C and maintained thereat, with stirring for about 24 hours. The reactor was opened to atmospheric pressure. Analysis of the liquid reaction product by F-19 nuclear magnetic resonance techniques indicated 7.7% of the starting anthraniloyl fluoride; 20.8% of 2-(trifluoromethyl)-benzenamine·hydrofluoride; and 71.5% of 2-(trifluoromethyl)phenyl carbamic fluoride.

*Examples 13-16*

The procedure of Example 12 was repeated except that various catalysts were incorporated and the molar proportion of hydrogen fluoride and N-(trifluoromethyl) anthraniloyl fluoride reactants was varied with the results as shown in Table 2, below. The analysis of the liquid reaction product, in each instance indicated the major component as 2-(trifluoromethyl)phenyl carbamic fluoride (Compound I) with minor proportions of 2-(trifluoromethyl)-benzenamine-hydrofluoride (Compound II) and N-(trifluoromethyl)anthraniloyl fluoride starting material.

TABLE 2

| Example | Mole Ratio HF:Organic[1] | Catalyst/Parts | Starting Material | Product Composition (%) Compound I[3] | Compound II[4] |
|---|---|---|---|---|---|
| 13 | 15.6 | None | 1.7 | 87.0 | 11.3 |
| 14 | 9.2 | $SbCl_5$/5 parts | 18.7 | 78.3 | 3.4 |
| 15 | 10.3 | $FSO_3H$/11 parts | 16.1 | 73.6 | 10.3 |
| 16 | 13.7 | HCl[2] | 7.4 | 79.6 | 13.0 |

[1]Mole Ratio of HF:N-(trifluoromethyl)-anthraniloyl fluoride
[2]HF used was saturated with HCl
[3]2-(trifluoromethyl)phenyl carbamic fluoride
[4]2-(trifluoromethyl)benzenamine hydrofluoride

*Examples 17-24*

*Preparation of N-(Trifluoromethyl)-Anthraniloyl Fluoride*

*Example 17*

Anhydrous hydrogen fluoride and 1-isocyanato-2-(trichloromethyl)-benzene were fed at rates of 24 parts per hour and 3 parts per hour, respectively, (mol ratio of HF: organic reactant = 94.66) into a vapor phase reactor maintained at about 275°C. The poduct gases werre cooled, condensed and collected. Analysis, of the reaction product, using F-19 nuclear magnetic resonance techniques, indicated an essentially 100% conversion of the organic reactant to N-(trifluoromethyl)-anthraniloyl fluoride.

*Examples 18-24*

The procedure of Example 12 was repeated except that the temperature of the reactor was varied and the flow rates of reactants were varied to provide molar rations as shown in the table below.

TABLE 3

| Example | Temperature | Molar Ratio[1] | Conversions[2] % |
|---|---|---|---|
| 18 | 300 | 21.29 | 100 |
| 19 | 300 | 94.60 | 100 |
| 20 | 300 | 48.76 | 96.1 |
| 21 | 275 | 35.48 | 96.2 |
| 22 | 275 | 65.04 | 100 |
| 23 | 250 | 94.62 | 100 |
| 24 | 250 | 26.61 | 97.0 |

[1]Molar Ratio of HF:1-isocyanato-2-(trichloromethyl)-benzene
[2]Percent conversion of 1-isocyanato-2-(trichloromethyl)-benzene to N-(trifluoromethyl)-anthraniloyl fluoride

**Claims**

1. A method for the preparation of benzenamine·hydrofluoride complexes of the formula

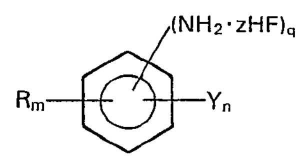

wherein R is chlorine, fluorine, bromine, phenyl, chlorophenyl, fluorophenyl, or bromophenyl; Y is trifluoromethyl or difluoromethyl; m is 0 to 2; n is 0 to 2; z is 1 to 4; and q is 1 to 2; characterized by reacting hydrogen fluoride with a phenyl carbamic fluoride of the fomula

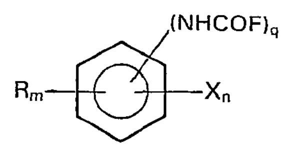

where q, m, n, and R are as defined above, and X is trichloromethyl, tribromomethyl, trifluoromethyl, dichloromethyl, dibromomethyl or difluoromethyl with the provision that when X is trichloromethyl, tribromomethyl or trifluoromethyl, Y is trifluoromethyl and when X is dichloromethyl, dibromomethyl or difluoromethyl, Y is difluoromethyl.

2. A method according to claim 1, for the preparation of benzenamine·hydrofluoride complexes of the formula

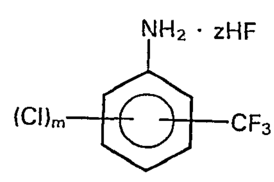

where m is 0 or 1, which comprises reacting hydrogen fluoride with a phenyl carbamic fluoride of the formula

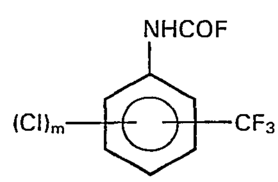

where m is as defined above.

3. A method according to claim 2 wherein the molar ratio of hydrogen fluoride:phenyl carbamic fluoride is 4:1 to 30:1 and the method is carried out at a temperature of 20° to 100° Celsius.

4. A method according to claim 2 wherein the phenyl carbamic fluoride is 2-(trifluoromethyl)phenyl carbamic fluoride and the benzenamine·hydrofluoride complex prepared is 2-(trifluoromethyl)benzenamine·hydrofluoride.

5. A method according to claim 2 wherein the phenyl carbamic fluoride is 3-(trifluoromethyl)phenyl carbamic fluoride and the benzenamine·hydrofluoride is 3-(trifluoromethyl)benzenamine·hydrofluoride.

6. A method according to claim 2 wherein the phenyl carbamic fluoride is 4-(trifluoromethyl)phenyl carbamic fluoride and the benzenamine·hydrofluoride complex prepared is 4-(trifluoromethyl)benzenamine·hydrofluoride.

7. A method according to claim 2 wherein the phenyl carbamic fluoride is 4-chloro-2-(trifluoromethyl)phenyl carbamic fluoride and the benzenamine·hydrofluoride complex prepared is 4-chloro-2-(trifluoromethyl)benzenamine·hydrofluoride.

8. A method according to claim 2 wherein the phenyl carbamic fluoride is 4-chloro-3-(trifluoromethyl)phenyl carbamic fluoride and the benzenamine·hydrofluoride complex prepared is 4-chloro-3-(trifluoromethyl)benzenamine·hydrofluoride.

9. A method according to claim 2 wherein a 2-(trifluoromethyl)benzenamine·hydrofluoride complex of the formula

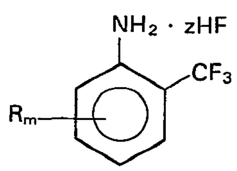

where m is 0 to 2 and R is independently selected from the group fluorine, chlorine, bromine, phenyl-, chlorophenyl-, fluorophenyl-, and bromophenyl-; is prepared by reacting hydrogen fluoride with a 2-(trifluoromethyl)phenyl carbamic fluoride of the formula

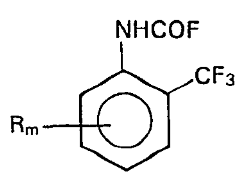

where m and R are as defined above.

10. A method according to claim 9 wherein the 2-(trifluoromethyl)phenyl carbamic fluoride is prepared in situ by isomerization of an N-(trifluoromethyl)anthraniloyl fluoride of the formula

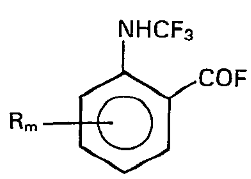

where m and R are as defined above.

11. A method according to claim 10 wherein the isomerization is carried out at a temperature of —10 to about 150° Celsius and in the presence of a molar ratio of hydrogenfluoride:N-(trifluoromethyl)anthraniloyl fluoride of at least about 1.5:1.

12. A method according to claim 11 wherein the N-(trifluoromethyl)anthraniloyl fluoride is prepared by the reaction of 1-isocyanato-2-(trichloromethyl)-benzene with hydrogen fluoride.

13. A method according to claim 2 wherein the phenyl carbamic fluoride is prepared in situ by the reaction of hydrogen fluoride with a phenyl isocyanate of the formula

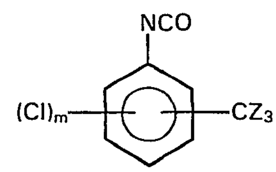

where m is as defined, and Z is halo.

14. A method according to claim 13 wherein m is 0.

15. A method according to claim 3 wherein water is present in an amount of about 0.001 to about 2.0 moles per mole of phenyl carbamic fluoride.

16. A method for the preparation of a benzenamine, characterized in that the benzenamine·hydrofluoride complex obtained according to claim 1 is dissociated and a benzenamine of the formula

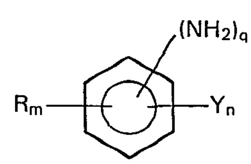

where R, Y, m, n and q are as defined, is recovered.

17. A method according to claim 16 wherein the reaction of hydrogen fluoride with a phenyl carbamic fluoride is carried out at a temperature of about —10° to about 150° Celsius.

18. A method according to claim 16 wherein the molar ratio of hydrogen fluoride:phenyl carbamic fluoride in step (A) is about 4:1 to about 30:1.

19. A method according to claim 16 wherein the phenyl carbamic fluoride is 2-(trifluoromethyl)phenyl carbamic fluoride and the benzenamine prepared is 2-(trifluoromethyl)benzenamine.

20. A method according to claim 16 wherein the phenyl carbamic fluoride is 3-(trifluoromethyl)phenyl carbamic fluoride and the benzenamine prepared is 3-(trifluoromethyl)benzenamine.

21. A method according to claim 16 wherein the phenyl carbamic fluoride is 4-(trifluoromethyl)phenyl carbamic fluoride and the benzenamine prepared is 4-(trifluoromethyl)benzenamine.

22. A method according to claim 16 wherein the phenyl carbamic fluoride is 4-chloro-2-(trifluoromethyl)phenyl carbamic fluoride and the benzenamine prepared is 4-chloro-2-(trifluoromethyl)benzenamine.

23. A method according to claim 16 wherein the phenyl carbamic fluoride is 4-chloro-3-(trifluoromethyl)phenyl carbamic fluoride and the benzenamine prepared is 4-chloro-3-(trifluoromethyl)benzenamine.

24. A method according to claim 16 wherein the phenyl carbamic fluoride is of the formula

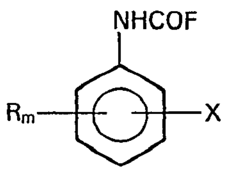

where R, m and X are as defined, and the benzenamine prepared is of the formula

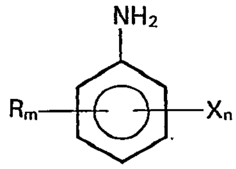

where X, R, m and n are as defined.

25. A method according to claim 24 wherein the phenyl carbamic fluoride is prepared in situ by the reaction of hydrogen fluoride with a phenyl isocyanate of the formula

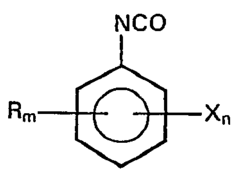

where X, R, m and n are as defined.

26. A method according to claim 19 wherein the 2-(trifluoromethyl)phenyl carbamic fluoride is prepared in situ by reaction of 1-isocyanato-2-(trifluoromethyl)benzene with hydrogen fluoride.

27. A method according to claim 20 wherein the 3-(trifluoromethyl)phenyl carbamic fluoride is prepared in situ by reaction of 1-isocyanato-3-(trifluoromethyl)benzene with hydrogen fluoride.

28. A method according to claim 21 wherein the 4-(trifluoromethyl)phenyl carbamic fluoride is prepared in situ by reaction of 1-isocyanato-4-(trifluoromethyl)benzene with hydrogen fluoride.

29. A method according to claim 22 wherein the 4-chloro-2-(trifluoromethyl)phenyl carbamic fluoride is prepared in situ by reaction of 4-chloro-2-(trifluoromethyl)isocyanatobenzene with hydrogen fluoride.

30. A method according to claim 23 wherein the 4-chloro-3-(trifluoromethyl)phenyl carbamic fluoride is prepared in situ by reaction of 4-chloro-3-(trifluoromethyl)isocyanatobenzene with hydrogen fluoride.

31. A method for the preparation of a 2-(trifluoromethyl)phenyl carbamic fluoride of the formula

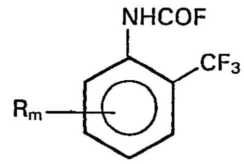

wherein m is 0 to 2 and R is independently selected from the group fluorine, chlorine, bromine, phenyl-, chlorophenyl-, fluorophenyl-, and bromophenyl-, which comprises isomerizing an N-(trifluoromethyl) anthraniloyl fluoride of the formula

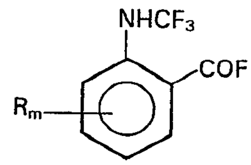

where m and R are as defined above in the presence of hydrogen fluoride.

32. A method according to claim 31 wherein the isomerization is carried out at a temperature of about —10° to 150° Celsius and in the presence of a molar ratio of hydrogen fluoride:N-(trifluoromethyl) anthraniloyl fluoride of greater than about 1.5:1.

33. A method according to claim 32 wherein m is 0.

**Patentansprüche**

1. Verfahren zur Herstellung von Benzolamin-Hydrofluorid-Komplexen der allgemeinen Formel

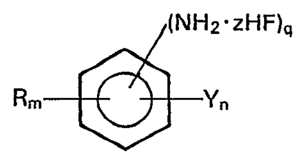

in der R ein Chlor-, Fluor- oder Bromatom- oder eine Phenyl-, Chlorphenyl-, Fluorphenyl- oder Bromphenylgruppe bedeutet, Y eine Trifluormethyl- oder Difluormethylgruppe darstellt, m den Wert 0 bis 2, n den Wert 0 bis 2, z den Wert 1 bis 4 und q den Wert 1 bis 2 aufweist, dadurch gekennzeichnet, dass man Fluorwasserstoff mit einem Phenylcarbaminfluorid der folgenden Formel umsetzt,

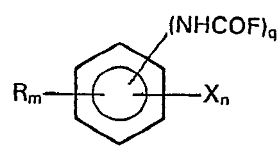

in der q, m, n und R die vorstehend angegebene Bedeutung haben, und X eine Trichlormethyl-, Tribrommethyl-, Trifluormethyl-, Dichlormethyl-, Dibrommethyl- oder Difluormethylgruppe bedeutet, mit der Massgabe, dass, wenn X eine Trichlormethyl-, Tribrommethyl- oder Trifluormethylgruppe ist,

Y eine Trifluormethylgruppe bedeutet, und wenn X eine Dichlormethyl-, Dibrommethyl- oder Difluormethylgruppe ist, Y eine Difluormethylgruppe bedeutet.

2. Verfahren nach Anspruch 1 zur Herstellung von Benzamin-Hydrofluorid-Komplexen der allgemeinen Formel

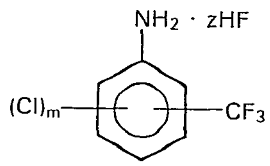

in der m den Wert 0 oder 1 hat, dadurch gekennzeichnet, dass man Fluorwasserstoff mit einem Phenylcarbaminfluorid der allgemeinen Formel

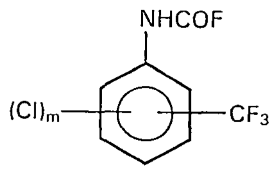

in der m die vorstehende angegebene Bedeutung hat, umsetzt.

3. Verfahren nach Anspruch 2, in dem das molare Verhältnis von Fluorwasserstoff zu Phenylcarbaminfluorid 4 : 1 bis 30 : 1 beträgt und das Verfahren bei einer Temperatur von 20 bis 100°C durchgeführt wird.

4. Verfahren nach Anspruch 2, in dem das Phenylcarbaminfluorid 2-(Trifluormethyl)-phenylcarbaminfluorid ist und der hergestellte Benzolamin-Hydrofluorid-Komplex 2-(Trifluormethyl)-benzolamin-hydrofluorid ist.

5. Verfahren nach Anspruch 2, in dem das Phenylcarbaminfluorid 3-(Trifluormethyl)-phenylcarbaminfluorid ist und das Benzolamin-Hydrofluorid 3-(Trifluormethyl)-benzolamin-hydrofluorid ist.

6. Verfahren nach Anspruch 2, in dem das Phenylcarbaminfluorid 4-(Trifluormethyl)-phenylcarbaminfluorid ist und der hergestellte Benzolamin-Hydrofluorid-Komplex 4-(Trifluormethyl)-benzolamin-hydrofluorid ist.

7. Verfahren nach Anspruch 2, in dem das Phenylcarbaminfluorid 4-Chlor-2-(trifluormethyl)-phenylcarbaminfluorid und der hergestellte Benzolamin-Hydrofluorid-Komplex 4-Chlor-2-(trifluormethyl)-benzolamin-hydrofluorid ist.

8. Verfahren nach Anspruch 2, in dem das Phenylcarbaminfluorid 4-Chlor-3-(trifluormethyl)-phenylcarbaminfluorid und der hergestellte Benzolamin-Hydrofluorid-Komplex 4-Chlor-3-(trifluormethyl)-benzolamin-hydrofluorid ist.

9. Verfahren nach Anspruch 2, in dem ein 2-(Trifluormethyl)-benzolamin-hydrofluorid-Komplex der allgemeinen Formel

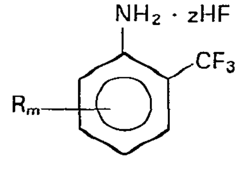

in der m den Wert 0 bis 2 aufweist und R unabhängig davon ein Fluor-, Chlor- oder Bromatom oder eine Phenyl-, Chlorphenyl-, Fluorphenyl- oder Bromphenylgruppe bedeutet, durch Umsetzung von Fluorwasserstoff mit einem 2-(Trifluormethyl)-phenylcarbaminfluorid der allgemeinen Formel

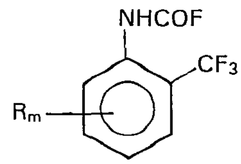

in der m und R die vorstehend angegebene Bedeutung haben, hergestellt wird.

10. Verfahren nach Anspruch 9, in dem das 2-(Trifluormethyl)-phenylcarbaminfluorid in situ durch Isomerisierung eines N-(Trifluormethyl)-anthraniloylfluorids der allgemeinen Formel

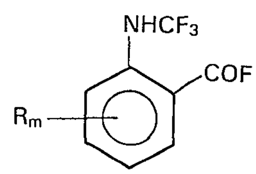

in der m und R die vorstehend angegebene Bedeutung haben, hergestellt wird.

11. Verfahren nach Anspruch 10, in dem die Isomerisierung bei einer Temperatur von —10 bis etwa 150°C und in Gegenwart eines molaren Verhältnisses von Fluorwasserstoff zu N-(Trifluormethyl)-anthraniloylfluorid von mindestens etwa 1,5 : 1 durchgeführt wird.

12. Verfahren nach Anspruch 11, in dem das N-(Trifluormethyl)-anthraniloylfluorid durch Umsetzung von 1-Isocyanato-2-(trichlormethyl)-benzol mit Fluorwasserstoff hergestellt wird.

13. Verfahren nach Anspruch 2, in dem das Phenylcarbaminfluorid in situ durch Umsetzung von Fluorwasserstoff mit einem Phenylisocyanat der allgemeinen Formel

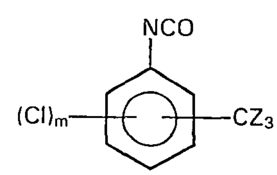

in der m die vorstehend angegebene Bedeutung hat und Z ein Halogenatom bedeutet, hergestellt wird.

14. Verfahren nach Anspruch 13, in dem m den Wert 0 hat.

15. Verfahren nach Anspruch 3, in dem Wasser in einer Menge von etwa 0,001 bis etwa 2,0 Mole pro Mol Phenylcarbaminfluorid vorliegt.

16. Verfahren zur Herstellung eines Benzolamins nach Anspruch 1, dadurch gekennzeichnet, dass der erhaltene Benzolamin-Hydrofluorid-Komplex dissoziiert wird und ein Benzolamin der allgemeinen Formel

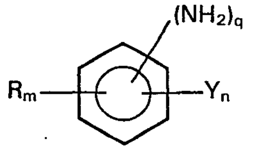

in der R, Y, m, n und q die vorstehend angegebene Bedeutung haben, gewonnen wird.

17. Verfahren nach Anspruch 16, in dem die Umsetzung von Fluorwasserstoff mit einem Phenylcarbaminfluorid bei einer Temperatur von etwa —10° bis etwa 150°C durchgeführt wird.

18. Verfahren nach Anspruch 16, in dem das molare Verhältnis von Fluorwasserstoff zu Phenylcarbaminfluorid in Schritt (A) etwa 4 : 1 bis 30 : 1 beträgt.

19. Verfahren nach Anspruch 16, in dem das Phenylcarbaminfluorid 2-(Trifluormethyl)-phenylcarbaminfluorid ist und das hergestellte Benzolamin 2-(Trifluormethyl)-benzolamin ist.

20. Verfahren nach Anspruch 16, in dem das Phenylcarbaminfluorid 3-(Trifluormethyl)-phenylcarbaminfluorid und das hergestellte Benzolamin 3-(Trifluormethyl)-benzolamin ist.

21. Verfahren nach Anspruch 16, in dem das Phenylcarbaminfluorid 4-(Trifluormethyl)-phenylcarbaminfluorid und das hergestellte Benzolamin 4-(Trifluormethyl)-benzolamin ist.

22. Verfahren nach Anspruch 16, in dem das Phenylcarbaminfluorid 4-Chlor-2-(trifluormethyl)-phenylcarbaminfluorid und das hergestellte Benzolamin 4-Chlor-2-(trifluormethyl)-benzolamin ist.

23. Verfahren nach Anspruch 16, in dem das Phenylcarbaminfluorid 4-Chlor-3-(trifluormethyl)-phenylcarbaminfluorid und das hergestellte Benzolamin 4-Chlor-3-(trifluormethyl)-benzolamin ist.

24. Verfahren nach Anspruch 16, in dem das Phenylcarbaminfluorid die folgende allgemeine Formel hat

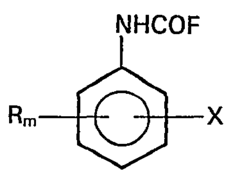

in der R, m und X die vorstehend angegebene Bedeutung haben, und das hergestellte Benzolamin die folgende allgemeine Formel hat

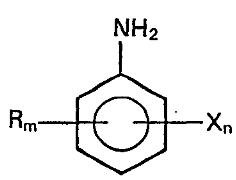

in der X, R, m und n die vorstehend angegebene Bedeutung haben.

25. Verfahren nach Anspruch 24, in dem das Phenylcarbaminfluorid in situ durch die Umsetzung von Fluorwasserstoff mit einem Phenylisocyanat der folgenden allgemeinen Formel hergestellt wird,

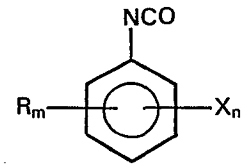

in der X, R, m und n die vorstehend angegebene Bedeutung haben.

26. Verfahren nach Anspruch 19, in dem das 2-(Trifluormethyl)-phenylcarbaminfluorid in situ durch Umsetzung von 1-Isocyanato-2-(trifluormethyl)-benzol mit Fluorwasserstoff hergestellt wird.

27. Verfahren nach Anspruch 20, in dem das 3-(Trifluormethyl)-phenylcarbaminfluorid in situ durch Umsetzung von 1-Isocyanato-3-(trifluormethyl)-benzol mit Fluorwasserstoff hergestellt wird.

28. Verfahren nach Anspruch 21, in dem das 4-(Trifluormethyl)-phenylcarbaminfluorid in situ durch Umsetzung von 1-Isocyanato-4-(trifluormethyl)-benzol mit Fluorwasserstoff hergestellt wird.

29. Verfahren nach Anspruch 22, in dem das 4-Chlor-2-(trifluormethyl)-phenylcarbaminfluorid in situ durch Umsetzung von 4-Chlor-2-(trifluomethyl)-isocyanatobenzol mit Fluorwasserstoff hergestellt wird.

30. Verfahren nach Anspruch 23, in dem das 4-Chlor-3-(trifluormethyl)-phenylcarbaminfluorid in situ durch Umsetzung von 4-Chlor-3-(trifluormethyl)-isocyanatobenzol mit Fluorwasserstoff hergestellt wird.

31. Verfahren zur Herstellung eines 2-(Trifluormethyl)-phenylcarbaminfluorids der allgemeinen Formel

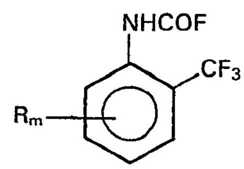

in der m den Wert 0 bis 2 hat und R unabhängig davon ein Fluor-, Chlor- oder Bromatom oder eine Phenyl-, Chlorphenyl-, Fluorphenyl- oder Bromphenylgruppe bedeutet, dadurch gekennzeichnet, dass man ein N-(Trifluormethyl)-anthraniloylfluorid der allgemeinen Formel

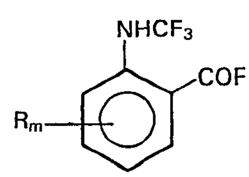

in der m und R die vorstehend angegebene Bedeutung haben, in Gegenwart von Fluorwasserstoff isomerisiert.

32. Verfahren nach Anspruch 31, in dem die Isomerisierung bei einer Temperatur etwa —10 bis etwa 150°C und in Gegenwart eines molaren Verhältnisses von Fluorwasserstoff zu N-(Trifluormethyl)-anthraniloylfluorid von mehr als etwa 1,5 : 1 durchgeführt wird.

33. Verfahren nach Anspruch 33, in dem m den Wert 0 hat.

**Revendications**

1. Procédé de préparation de complexes de fluorhydrate de benzèneamine de formule:

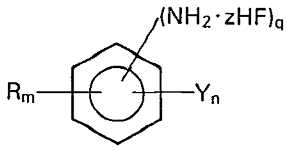

dans laquelle R est un atome de chlore, de fluor ou de brome ou un radical phényle, chlorophényle, fluorophényle, ou bromophényle; Y est le radical trifluorométhyle ou difluorométhyle; m est un nombre de 0 à 2; n est un nombre de 0 à 2; z est un nombre de 1 à 4 et q est un nombre de 1 à 2; caractérisé en ce qu'on fait réagir de l'acide fluorhydrique avec un fluorure phénylcarbamique de formule:

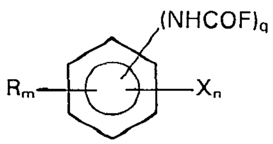

dans laquelle q, m, n et R sont tels que définis plus haut et X est un radical trichlorométhyle, tribromométhyle, trifluorométhyle, dichlorométhyle, dibromométhyle ou difluorométhyle, à la condition que quand X est le radical trichlorométhyle, tribromométhyle ou trifluorométhyle, Y est le radical trifluorométhyle et quand X est le radical dichlorométhyle, dibromométhyle ou difluorométhyle, Y est le radical difluorométhyle.

2. Procédé selon la revendication 1 pour la préparation de complexes de fluorhydrate de benzèneamine de formule:

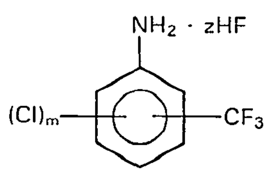

dans laquelle m est 0 ou 1, qui comprend le fait de faire réagir de l'acide fluorhydrique avec un fluorure phénylcarbamique de formule:

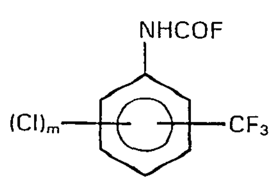

dans laquelle m est tel que défini ci-dessus.

3. Procédé selon la revendication 2, dans lequel le rapport molaire acide fluorhydrique : fluorure phénylcarbamique est de 4 : 1 à 30 : 1 et on effectue le procédé à une température de 20 à 100°C.

4. Procédé selon la revendication 2, dans lequel le fluorure phénylcarbamique est le fluorure 2-(trifluorométhyl)phénylique carbamique et le complexe de fluorhydrate de benzèneamine préparé est le fluorhydrate de 2-(trifluorométhyl)benzèneamine.

5. Procédé selon la revendication 2, dans lequel le fluorure phénylcarbamique est le fluorure 3-(trifluorométhyl)phénylcarbamique et le fluorhydrate de benzèneamine est le fluorhydrate de 3-(trifluorométhyl)benzèneamine.

6. Procédé selon la revendication 2, dans lequel le fluorure phénylcarbamique est le fluorure 4-(trifluorométhyl)phénylcarbamique et le complexe de fluorhydate de benzèneamine préparé est le fluorhydrate de 4-(trifluorométhyl)benzèneamine.

7. Procédé selon la revendication 2, dans lequel le fluorure phénylcarbamique est le fluorure 4-chloro-2-(trifluorométhyl)phénylcarbamique et le complexe de fluorhydrate de benzèneamine préparé est le fluorhydrate de 4-chloro-2-(trifluorométhyl)benzèneamine.

8. Procédé selon la revendication 2, dans lequel le fluorure phénylcarbamique est le fluorure 4-chloro-3-(trifluorométhyl)phénylcarbamique et le complexe de fluorhydrate de benzèneamine préparé est le fluorhydrate de 4-chlor-3-(trifluorométhyl)benzèneamine.

9. Procédé selon la revendication 2, dans lequel on prépare le complexe de fluorhydrate de 2-(trifluorométhyl)benzèneamine de formule:

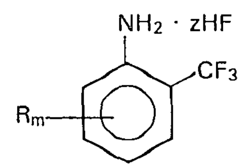

dans laquelle m est 0 à 2 et R est choisi indépendamment parmi le fluor, le chlore, le brome, le phényle, le chlorophényle, le fluorophényle et le bromophényle, en faisant réagir l'acide fluorhydrique avec un fluorure 2-(trifluorométhyl)phénylcarbamique de formule:

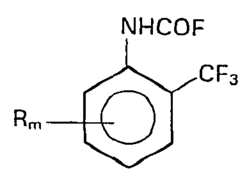

dans laquelle m et R sont tels que définis plus haut.

10. Procédé selon la revendication 9, dans lequel on prépare le fluorure 2-(trifluorométhyl)phénylcarbamique in situ par isomérisation d'un fluorure de N-(trifluorométhyl)anthraniloyle de formule:

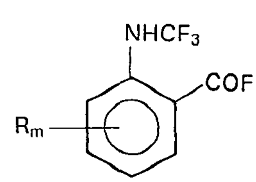

dans laquelle m et R sont tels que définis plus haut.

11. Procédé selon la revendication 10, dans lequel on effectue l'isomérisation à une température de —10 à environ 150°C et avec un rapport molaire acide fluorhydride : fluorure de N-(trifluorométhyl) anthraniloyle d'au moins 1,5 : environ.

12. Procédé selon la revendication 11, dans lequel on prépare le fluorure de N-(trifluorométhyl) anthraniloyle par réaction du 1-isocyanato-2-(trichlorométhyl)benzène avec l'acide fluorhydrique.

13. Procédé selon la revendication 2, dans lequel on prépare le fluorure phénylcarbamique in situ par réaction d'acide fluorhydrique avec un isocyanate de phényle de formule:

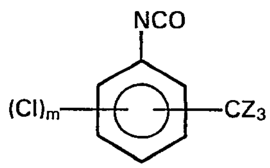

dans laquelle m est tel que défini et Z est un radical halo.

14. Procédé selon la revendication 13, dans lequel m est 0.

15. Procédé selon la revendication 3, dans lequel l'eau est présente à raison d'environ 0,001 à environ 2,0 moles par mole de fluorure phénylcarbamique.

16. Procédé pour la préparation d'une benzèneamine, caractérisé en ce qu'on dissocie le complexe de fluorhydrate de benzèneamine obtenu selon la revendication 1 et on récupère une benzèneamine de formule:

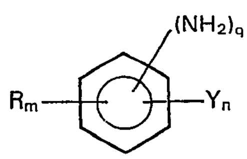

dans laquelle R, Y, m, n et q sont tels que définis.

17. Procédé selon la revendication 16, dans lequel on effectue la réaction de l'acide fluorhydrique avec un fluorure phénylcarbamique à une température d'environ —10 à 150°C.

18. Procédé selon la revendication 16, dans lequel le rapport molaire acide fluorhydrique : fluorure phénylcarbamique dans l'étape (A) est d'environ 4 : 1 à 30 : 1.

19. Procédé selon la revendication 16, dans lequel le fluorure phénylcarbamique est le fluorure 2-(trifluorométhyl)phénylcarbamique et la benzèneamine préparée est la 2-(trifluorométhyl)benzèneamine.

20. Procédé selon la revendication 16, dans lequel le fluorure phénylcarbamique est le fluorure 3-(trifluorométhyl)phénylcarbamique et la benzèneamine préparée est la 3-(trifluorométhyl)benzèneamine.

21. Procédé selon la revendication 16, dans lequel le fluorure phénylcarbamique est le fluorure 4-(trifluorométhyl)phénylcarbamique et la benzèneamine préparée est la 4-(trifluorométhyl)benzèneamine.

22. Procédé selon la revendication 16, dans lequel le fluorure phénylcarbamique est le fluorure 4-chloro-2-(trifluorométhyl)phénylcarbamique et la benzèneamine préparée est la 4-chloro-2-(trifluorométhyl)benzèneamine.

23. Procédé selon la revendication 16, dans lequel le fluorure phénylcarbamique est le fluorure 4-chloro-3-(trifluorométhyl)phénylcarbamique et la benzèneamine préparée est la 4-chloro-3-(trifluorométhyl)benzèneamine.

24. Procédé selon la revendication 16, dans lequel le fluorure phénylcarbamique est de formule:

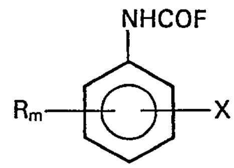

dans laquelle R, m et X sont tels que définis, et la benzèneamine préparée répond à la formule:

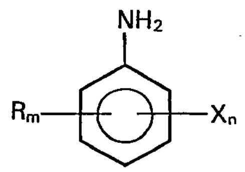

dans laquelle X, R, m et n sont tels que définis.

25. Procédé selon la revendication 24, dans lequel on prépare le fluorure phénylcarbamique in situ par réaction d'acide fluorhydrique avec un isocyanate de phényle de formule:

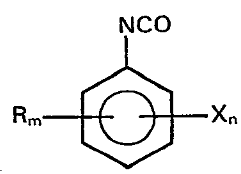

dans laquelle X, R, m et n sont tels que définis.

26. Procédé selon la revendication 19, dans lequel on prépare in situ le fluorure 2-(trifluorométhyl)-phénylcarbamique par réaction de 1-isocyanato-2-(trifluorométhyl)benzène avec de l'acide fluorhydrique.

27. Procédé selon la revendication 20, dans lequel on prépare in situ le fluorure 3-(trifluorométhyl)phénylcarbamique par réaction de 1-isocyanato-3-(trifluorométhyl)benzène avec de l'acide fluorhydrique.

28. Procédé selon la revendication 21, dans lequel on prépare in situ le fluorure 4-(trifluorométhyl)phénylcarbamique par réaction de 1-isocyanato-4-(trifluorométhyl)benzène avec de l'acide fluorhydrique.

29. Procédé selon la revendication 22, dans lequel on pépare in situ le fluorure 4-chloro-2-(trifluorométhyl)phénylcarbamique par réaction de 4-chlor-2-(trifluorométhyl)isocyanatobenzène avec de l'acide fluorhydrique.

30. Procédé selon la revendication 23, dans lequel on prépare in situ le fluorure 4-chloro-3-(trifluorométhyl)phénylcarbamique par réaction de 4-chlor-3-(trifluorométhyl)isocyanatobenzène avec l'acide fluorhydrique.

31. Procédé pour la préparation de fluorure 2-(trifluorométhyl)phénylcarbamique de formule:

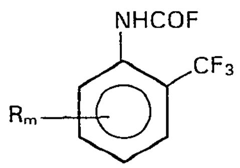

dans laquelle m est 0 à 2, et R est choisi indépendamment parmi le fluor, le chlore, le brome, le phényle, le chlorophényle, le fluorophényle et le bromophényle, qui comprend le fait d'isomériser un fluorure de N-(trifluorométhyl)anthraniloyle de formule:

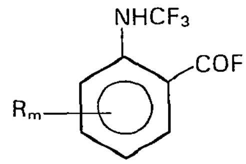

dans laquelle m et R sont tels que définis plus haut en présence d'acide fluorhydrique.

32. Procédé selon la revendication 31, dans lequel on effectue l'isomérisation à une température d'environ —10 à environ 150°C et avec un rapport molaire d'acide fluorhydrique : fluorure de N-(trifluorométhyl)anthraniloyle supérieur à environ 1,5 : 1.

33. Procédé selon la revendication 32, dans lequel m est 0.